# EUROPEAN PATENT SPECIFICATION

(11) **EP 4 201 470 B1**
(45) Date of publication and mention of the grant of the patent: **13.08.2025**
(21) Application number: 21217497.3
(22) Date of filing: 23.12.2021
(51) Int. Cl.: A61N 1/05, A61B 5/291, A61B 5/293, A61B 5/00

(54) **METHOD FOR MANUFACTURING AN ELECTRODE**
VERFAHREN ZUR HERSTELLUNG EINER ELEKTRODE
PROCÉDÉ DE FABRICATION D'UNE ÉLECTRODE

(43) Date of publication of application: 28.06.2023
(73) Proprietor: Imec VZW, 3001 Leuven (BE); Universiteit Gent, 9000 Gent (BE)
(72) Inventor: NIKOLAYEV, Denys, 79005 Lviv (UA); JOSEPH, Wout, 8570 Vichte (BE); MARTENS, Luc, 9840 De Pinte (BE); ANDREI, Alexandru, 1150 Sint-Pieters-Woluwe (BE); MORA LOPEZ, Carolina, 3010 Kessel-Lo (BE); TANGHE, Emmeric, 9000 Gent (BE)
(74) Representative: Winger

(56) References cited:
- WO-A1-2021/026662
- US-A1- 2019 030 318
- US-A1- 2021 085 961
- NIKOLAYEV DENYS ET AL: "Proceedings #63: Low-Profile 3D Microelectrodes with Near-Uniform Current Density for High-Resolution Neural Stimulation", BRAIN STIMULATION, vol. 12, no. 4, 2019, XP085718423, ISSN: 1935-861X, DOI: 10.1016/J.BRS.2019.03.021

## Description

### Technical field of the invention

The present invention relates to the field of electrode fabrication, and more in particular to the fabrication of microelectrodes.

### Background of the invention

Deep brain stimulation (DBS) is a technique used to treat neural disorders such as Parkinson's disease, tremor, and dystonia. A DBS system comprises tiny microelectrodes that are implanted in the brain to deliver stimulation pulses to the tissue, and an electrical pulse generator that generates the stimulation pulses and is connected to the electrodes.

Using electrodes to stimulate a live brain entails safety risks. In particular, a localized current of too high an intensity can cause damages to the brain tissues and/or to the electrode itself. For instance, this can cause corrosion of the electrode and undesired ions to enter the brain. Current density should therefore remain under a certain safety threshold. However, intensity should be high enough for the electrode to fulfil its function. It is, therefore, particularly undesirable for such electrodes to have a very uneven current density profile across their surface. Indeed, this typically translates in some area of the electrode not delivering enough current and some other area delivering too much of it. The edges of the electrode are typically problematic in this respect. It has been shown that the current density in planar electrodes is not uniform and is higher around the perimeter. Hence, for a given total current, a current density profile as flat as possible is desired. Injected charge is the time integral of the current, thus the main benefit of providing a current density as uniform as possible is that it is possible to achieve a higher safe injected charge

In theory, electrode shapes are known that can fulfil this need. In particular, a perfect sphere, a perfect half sphere above an infinite insulator, or a perfect disc infinitely recessed into an insulator should have a flat current density profile. In practice, however, an infinite insulator cannot be produced and a perfect sphere, a perfect half sphere, or a perfect disc cannot be achieved. Also, an electrode supported by a very large insulator could anyway not be used. Also an infinitively deep insulating well cannot be produced, and an electrode buried that way could anyway not be used.

Furthermore, the fabrication process should be easily scalable to mass manufacturing levels. Ideally, the fabrication should be compatible with CMOS processes.

Nilolayev Denys et al "Proceeding #63, brain stimulation, vol. 12, no. 4, 2019 determines an optimal design for an electrode.

WO 2021/026662 A1 discloses a manufacturing method for forming DBS electrode surrounded by an insulation layer.

There is therefore a need in the art for new manufacturing methods overcoming at least partially one or more of these issues.

### Summary of the invention

It is an object of the present invention to provide good electrodes and methods for manufacturing the same.

The above objective is accomplished by a method according to claim 1.

The present invention provides a method for manufacturing an electrode as defined in claim 1, the method for manufacturing an electrode, comprising the steps of:
a. providing an electrically conductive element over a top surface of a substrate,
b. measuring at least one parameter indicative of the shape or of the dimensions of the electrically conductive element,
c. simulating the electrically conductive element and a dielectric wall surrounding the same for a plurality of wall heights by using the at least one parameter as an input,
d. for each wall height, compute the maximum current density present at a surface of the electrically conductive element,
e. determine, from the maximum current densities computed in step d, one or more wall heights for which the maximum current density is below a predefined threshold,
f. providing an electrically conductive element, identical to the electrically conductive element provided in step a, surrounded by a wall having a wall height as determined in step e.

Embodiments of the present invention are defined by the dependent claims.

### SUMMARY OF THE PRESENT DISCLOSURE

The present disclosure relates to an electrode obtainable by the method of the present invention, the electrode comprising an electrically conductive element on a substrate, the electrically conductive element being surrounded by a wall which height is smaller than 0.1038 times, preferably smaller than 0.1000 times the length (or diameter) of the electrically conductive element, wherein said wall height (h) is measured from a bottom surface of the electrically conductive element to a top surface of the wall..

The present disclosure also relates to a brain stimulation system comprising such an electrode and an electrical pulse generator electrically coupled to the electrode

The present disclosure also relates to an array of said electrodes. electrodes

The present disclosure also relates to a neural probe comprising said array of electrodes.

It is an advantage of embodiments of the method of the present invention that it is CMOS compatible and, hence, scalable.

It is an advantage of embodiments of the method of the present invention that it makes use of standard processing steps without requiring complex or expensive techniques.

It is an advantage of embodiments of the method of the present invention that it allows the production of electrodes having an acceptable, or even optimized, current density profile in spite of an unavoidable imperfect electrode shape.

It is an advantage of embodiments of the method of the present invention that it makes use of actually produced electrodes to improve their current density profile in a tailor-made fashion.

It is an advantage of embodiments of the method of the present invention that it allows making electrodes achieving high safe injected charge.

It is an advantage of embodiments of the present invention that it makes DBS more easily achieved, safer, more precise, and more affordable.

It is an advantage of embodiments of the present invention that it permits the fabrication of electrodes having an acceptable current density profile while simultaneously comprising a surrounding wall which height is smaller than 0.1038 times, preferably smaller than 0.1000 times the length (or diameter) of the electrically conductive element, hence walls that can be easily fabricated and that do not hamper the use of the electrode.

The present invention is defined by the appended claims.

Although there has been constant improvement, change, and evolution of methods to produce devices in this field, the present concepts are believed to represent substantial new improvements, including departures from prior practices, resulting in the provision of more efficient, safe, stable, and reliable devices of this nature.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention which is defined by the appended claims. The reference figures quoted below refer to the attached drawings.

### Brief description of the drawings

Fig. 1 is a schematic representation of a process for forming electrodes in steps within embodiments of the present invention.
Fig. 2 is a schematic representation of another process for forming electrodes in steps within embodiments of the present invention.
Fig. 3 is a flow-chart summarizing the first aspect of the present invention.

In the different figures, the same reference signs refer to the same or analogous elements.

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third, and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter, it does not exclude other elements or steps. It is thus to be interpreted as specifying the presence of the stated features, integers, steps or components as referred to, but does not preclude the presence or addition of one or more other features, integers, steps or components, or groups thereof. The term "comprising" therefore covers the situation where only the stated features are present (and can therefore always be replaced by "consisting of" in order to restrict the scope to said stated features) and the situation where these features and one or more other features are present. The word "comprising" according to the invention therefore also includes as one embodiment that no further components are present. Thus, the scope of the expression "a device comprising means A and B" should not be interpreted as being limited to devices consisting only of components A and B. It means that with respect to the present invention, the only relevant components of the device are A and B.

Reference throughout this specification to "one embodiment" or "an embodiment" means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment, but may.

Furthermore, some of the embodiments are described herein as a method or combination of elements of a method can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

The invention will now be described by a detailed description of several embodiments of the invention. It is clear that other embodiments of the invention can be configured according to the knowledge of persons skilled in the art without departing from the technical teaching of the invention, the invention being limited only by the terms of the appended claims.

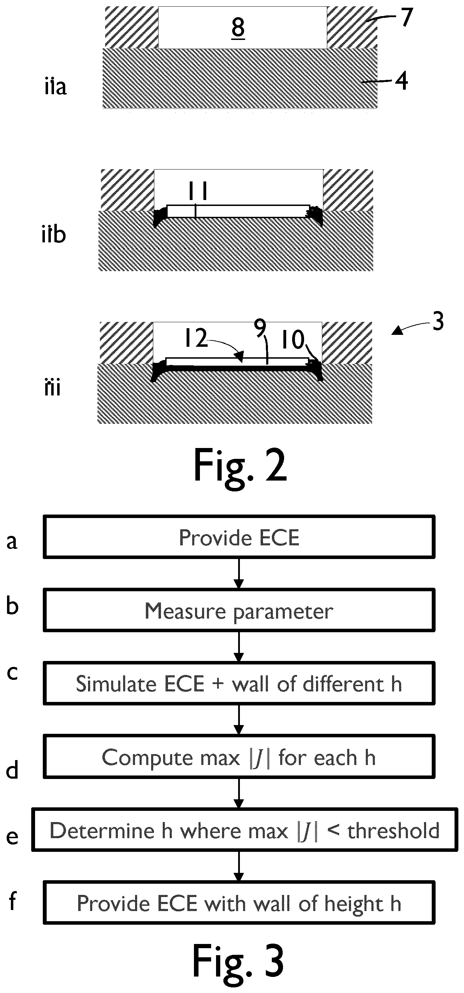

We now refer to Fig. 3.

The present invention relates to a method for manufacturing an electrode (3), comprising the steps of:
a. providing an electrically conductive element (ECE) (1) over a top surface of a substrate (5),
b. measuring at least one parameter indicative of the shape or of the dimensions of the electrically conductive element (1),
c. simulating the electrically conductive element (1) and a dielectric wall (2) surrounding the same for a plurality of wall heights (h) by using the at least one parameter as an input,
d. for each wall height (h), compute the maximum current density |*J*| present at a surface of the electrically conductive element (1),
e. determine, from the maximum current densities |*J*| computed in step d, one or more wall heights (h) for which the maximum current density |*J*| is below a predefined threshold,
f. providing an electrically conductive element (1), identical to the electrically conductive element (1) provided in step a, surrounded by a wall (2) having a wall height (h) as determined in step e.

Steps a to f are typically performed in that order.

In embodiments, step e may consist in identifying amongst the one or more wall heights (h) for which the maximum current density is below a predefined threshold, the wall height (h) giving rise to the lowest maximum current density, and wherein step f consists in providing an electrically conductive element (1), identical to the electrically conductive element (1) provided in step a, surrounded by a wall (2) having a wall height (h) closer to the wall height identified in step e than to any other wall heights (h) amongst said one or more wall heights (h).

In embodiments, the wall (2) surrounding the electrically conductive element (1) provided in step f may have a wall height (h) within 10% of a wall height (h) giving rise to the lowest maximum current density.

The electrode (3) is typically for use in brain stimulation, e.g., deep brain stimulation, or for use in in vitro cell tissue stimulation. However, the present invention also enables the fabrication of electrodes for use in batteries or galvanic cells.

The shape of the electrically conductive element (1) is not critical for the method of the present invention. In fact, an advantage of the method of the present invention is that it is adapted to any shape. However, some shapes have a flatter current density profile than others. These shapes are preferred.

In embodiments, a top surface of the electrically conductive element (1) provided in step a may have a at least a section having concave shape with a concavity facing the substrate (5). For instance, a preferred shape for the electrically conductive element (1) is a dome, i.e., a spheroid cap such as a spherical cap, preferably a half spheroid, and ideally a half-sphere.

In other embodiments, a top surface of the electrically conductive element (1) provided in step a may be flat while side walls of the electrically conductive element may have a concave shape with a concavity facing the substrate (5).

For instance, the electrically conductive element (1) may have the shape of a spheroid segment, preferably a spherical segment.

In embodiments, a geometrical centre of the top surface of the electrically conductive element (1) may be higher than any edge of the top surface of that electrically conductive element (1).

Preferably, the height on width ratio of the electrically conductive element (1) is 1 or lower, more preferably 0.5 of lower, wherein the height is measured perpendicular to the substrate and the width is the smallest dimension measured parallel to the substrate.

In embodiments, the electrically conductive element (1) provided in step a may have a length (or diameter) measured parallel to the top surface of the substrate (5) which is larger than a height measured perpendicular to the top surface of the substrate (5).

In embodiments, the electrically conductive element (1) provided in step a may have a length (or diameter) measured parallel to the top surface of the substrate (5) which measures from 10 to 200 µm, preferably from 25 to 150 µm, yet more preferably from 25 to 90 µm.

In embodiments, the electrically conductive element (1) provided in step a may have a height (H) measured perpendicular to the substrate equal to from 1 to 10 µm.

In embodiments, fabricating an electrically conductive element (1) during step a having a dome shape can be performed by first forming on the substrate (5) an electrically conductive element (1) having a different initial shape, e.g., a cylinder or a polyhedron such as a rectangle or a cube. This electrically conductive element (1) is, in this case, a solid homogeneous element (i.e., not a core-shell structure). Second, the electrically conductive element (1) can be heated up, thereby inducing the reflow of the initial shape, thereby forming a dome shaped conductive element. The temperature necessary for the reflow is however typically very high. This method is therefore not the most preferred.

The electrically conductive element (1) can be entirely made of one or more electrically conductive materials, or it can be made of a non-electrically conductive core (11) and a conductive shell (12).

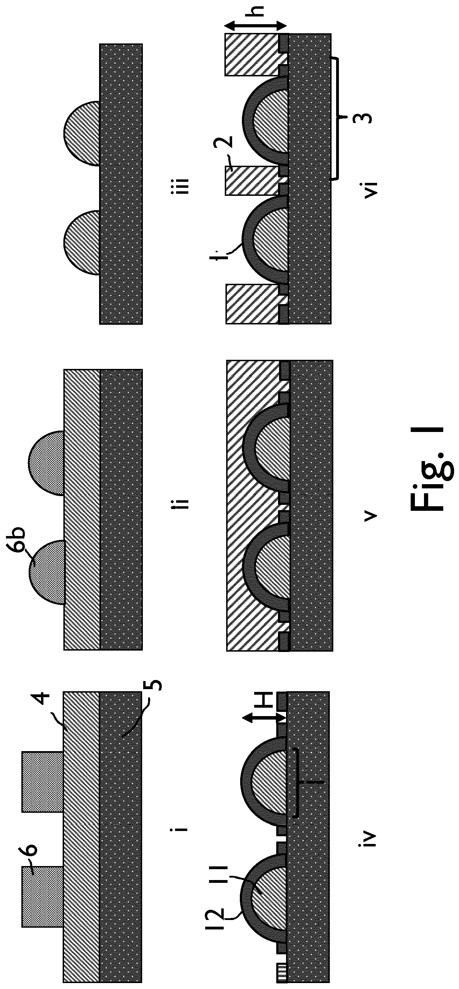

Preferably, a core-shell electrically conductive element (1) can be formed. Examples are depicted in Figs. 1 and 2.

In embodiments, the electrically conductive element (1) provided in step a may comprise a non-conductive core (11) in physical contact with the substrate (5) and projecting therefrom, and an electrically conductive layer (12) plating a top surface of the non-conductive core (11).

In embodiments, step a may comprise the steps of:
i. Providing a non-conductive layer (4),
ii. Patterning the non-conductive layer (4) to form a non-conductive core (11) projecting upward, and
iii. plating an electrically conductive layer (12) on the non-conductive core (11), thereby forming an electrically conductive element (1) comprising the non-conductive core (11) and the electrically conductive layer (12).

By non-conductive layer (4), it is meant a layer which is not electrically conductive.

For instance, the substrate (5) itself can be the non-conductive layer (4). In other embodiments, the non-conductive layer (4) may be over a substrate. In such a case, the non-conductive layer (4) is preferably a dielectric layer (4) such as a SiO₂ layer or an Si₃N₄ layer.

The non-conductive layer (4) preferably withstand at least 100°C, preferably at least 150°C without losing its physical integrity. This is advantageous because it allows the use of techniques such as electrodeposition for step iii.

In typical embodiments, step a may comprise the steps of:
i. Providing a non-conductive layer (4) over the substrate (5),
ii. Patterning the non-conductive layer (4) to form a non-conductive core (11) over the substrate (5) and projecting therefrom, and
iii. plating an electrically conductive layer (12) on the non-conductive core (11), thereby forming an electrically conductive element (1) comprising the non-conductive core (11) and the electrically conductive layer (12).

In embodiments, step ii may comprise the steps of:
ii.a. providing a further layer on the non-conductive layer (4),
ii.b. patterning the further layer to form a core shape (6b) in physical contact with the non-conductive layer (4) and projecting therefrom, and
ii.c. transferring the core shape (6b) into the non-conductive layer (4).

In embodiments, step ii.b. may comprise the steps of:
ii.b.i. patterning the further layer to form a precursor shape (6) in physical contact with the non-conductive layer (4) and projecting therefrom, and
ii.b.ii. melting the precursor shape (6), thereby forming the core shape (6b), wherein the core shape (6b) is shaped as a dome.

In embodiments, the further layer may be a photoresist.

In embodiments, the precursor shape (6) may be any shape. For instance, it can be a cylinder, or a polyhedron such as a rectangle or a cube. Preferably, it is a cylinder.

In embodiments, step ii.c. may be performed by a dry etching process, preferably reactive ion etching. Step ii.c. is typically performed until the further layer is fully etched, thereby entirely transferring its shape into the non-conductive layer (4).

In embodiments, the material forming the non-conductive layer (4) may have an etching rate with respect to the performed etching which is within 10%, preferably within 5%, yet more preferably which is the same as the etching rate of the photoresist material forming the photoresist element (6). This is advantageous as it allows the shape of the photoresist element (6) to be transferred to the non-conductive layer (4) with a relatively high fidelity. If the material forming the non-conductive layer (4) has an etching rate which is different from the etching rate of the photoresist element (6), the transfer of the shape of the photoresist element (6) into the dielectric layer (4) will be accompanied by a distortion. Although it is possible to take this distortion into account to arrive at a desired shape for the etched non-conductive layer (11), it is easier to select materials for the photoresist element (6) and the dielectric layer (4) that have a similar etching rate.

If the electrically conductive layer (12) is made of a metal, step iii can be performed by any suitable method such as CVD, PVD, sputtering, or electrodeposition. If the electrically conductive layer (12) is made of a ceramic, for instance TiN, it can be deposited by any suitable method such as CVD, PVD, or sputtering.

In an exemplary embodiment illustrated by Fig. 1 (i-iv), fabricating an electrically conductive element (1) having a dome shape can be performed by the steps of:
- Providing a dielectric layer (4) (e.g., on a substrate (5)),
- Forming (Fig. 1 i) on the substrate (5) a photoresist element (6) having an initial shape which is not a dome, e.g., a cylinder, or a polyhedron such as a rectangle or a cube,
- heating up (Fig. 1 ii) the photoresist element (6), thereby inducing reflowing of the initial shape, thereby forming a dome-shaped photoresist element (6b),
- etching (Fig. 1 iii) the dielectric layer (4) by using the dome-shaped photoresist element (6b) as a mask, thereby transferring the shape of the dome-shaped photoresist element (6b) into the dielectric layer (4) (this step can, for instance, be performed by reactive ion etching), and
- depositing (Fig. 1 iv) a layer of a conductive material conformally on the etched dielectric layer (4).

We now refer to Fig. 2 where an embodiment of the method to form an electrode (3) is depicted wherein a top surface of the electrically conductive element (1) provided in step a is flat while side walls of the electrically conductive element (1) have a concave shape with a concavity facing downward. In this embodiments, dielectric walls (2) are fabricated at the same time as the electrically conductive element (1).

In this embodiment, step a and/or step f may comprise the steps of:
i. Providing a non-conductive layer (4),
ii. Patterning the non-conductive layer (4) to form a non-conductive core (11) projecting upward by:
   iia. Providing a dielectric hard mask (7) on the non-conductive layer (4), the dielectric hard mask (7) having an opening (8), and
   iib. Dry etching the non-conductive layer (4) selectively with respect to the dielectric hard mask (7), thereby recessing the non-conductive layer (4) faster closer to the dielectric hard mask (7) than farther from it,
iii. plating an electrically conductive layer (12) on the non-conductive core (11), thereby forming an electrically conductive element (1) comprising the non-conductive core (11) and the electrically conductive layer (12), the electrically conductive element (1) having a flat top surface (9) and side walls (10) having a concave shape with a concavity facing downward.

Without being bound by theory, it is believed that the etching in step iib is faster closer to the dielectric hard mask (7) because the concentration in etching species is typically larger there.

In this embodiment, preferably, the non-conductive layer (4) is etched at least 10 times faster than the dielectric hard mask (7).

In this embodiment, if performed in step a, a certain height of the dielectric hard mask is already present, forming a precursor to the walls (2). In step f, this height can be adapted by either removing a top portion of the dielectric hard mask (7) or by adding a portion to the top surface of the dielectric hard mask (7).

The opening (8) is preferably round.

In embodiments, as for instance depicted in Fig. 1, a plurality of electrically conductive elements (1) can be formed by replacing the non-conductive core (11) by a plurality of non-conductive core (11) in step a. This can, for instance, be achieved by replacing the precursor shape (6) of step a by a plurality of precursor shapes (6). This way, after deposition of a layer of an electrically conductive material (12) conformally on the etched dielectric layer (11), a continuous electrically conductive layer (12) comprising a plurality of dome-shaped elements is produced. Next, the plurality of dome-shaped elements can be separated from one another, thereby forming the plurality of electrically conductive elements (1).

In embodiments, the method may be for manufacturing an array of electrodes (3), wherein step f comprises providing an array of electrically conductive elements (1), each conductive element being identical to the electrically conductive element (1) provided in step a, each conductive element being surrounded by a wall (2) having a wall height (h) as identified in step e.

In embodiments, the substrate (5) may be a shank at least 3 times, preferably at least 10 times, more preferably at least 50 times, yet more preferably at least 140 times as long as it is wide. As used herein, and unless provided otherwise, a shank is a straight object, or a straight part of an object, which is longer than it is wide. The length of the shank is its longest dimension, and it extends from one extremity of the shank to a second extremity. The shank has a flat surface running parallel to its length.. The electrically conductive element may be on that flat surface.

In embodiments, the width of the substrate (e.g. shank) may be from 12 to 250 µm, preferably from 50 to 125 µm, more preferably from 70 to 125 µm.

In embodiments, the length (or diameter) of the electrode, measured parallel to the top surface of the substrate, may be from 40 to 70% of the width of the substrate (e.g. shank).

The material of the substrate (5) is typically a non-electrically conductive material such as a dielectric material or an intrinsic semiconductor (e.g. Si).

Example of parameters for step b are the diameter, the height, and the curvature. Preferably the diameter, the height, and the curvature are all measured.

Step b can for instance be performed via a profilometry.

In embodiments, step b can be performed by scanning electron microscopy. This method has the advantage of a very high accuracy. However, it requires preparing a cross-section of the electrically conductive element (1). This method is therefore time consuming and cannot be applied in line. Also, it excludes the first main embodiment for performing step f (see infra).

In embodiments, step b can be performed by a 3D optical profiler such as an interferometer. Such devices are available, for instance, from Bruker. Such devices are advantageous as they allow step b to be performed in line, i.e., without having to cut into the electrically conductive element (1) provided in step a.

In steps c and f, the dielectric wall (2) is surrounding the electrically conductive element (1). It is typically surrounding it on all lateral sides. It leaves at least part of the top surface of the electrically conductive element (1) uncovered. This can be achieved by having both, the electrically conductive element (1) and the wall (2), on the substrate. In that case, the height of the wall (2) can for instance be measured from the top surface of the substrate or, equivalently, from the bottom surface of the electrically conductive element (1) with a same result. It can also be achieved by having the electrically conductive element (1) buried in the dielectric material forming the wall (2), the dielectric material forming a layer on the substrate (5). In this case, the height of the wall (2) is preferably the height of the dielectric material measured from the bottom surface of the electrically conductive element (1) to the top of the dielectric material forming the wall (2). In general, it is therefore preferred to measure the wall height (h) from the bottom surface of the electrically conductive element (1) to the top of the dielectric material forming the wall (2). The way the wall height is measured has, however, no impact on the method as long as it is measured in the same fashion in both steps c and f.

In steps c and f, the dielectric wall (2) is preferably touching the electrically conductive element (1). The dielectric wall (2) can for instance be next to the electrically conductive element (1), and touching it without overlapping with it, or it can overlap with the electrically conductive element (1) while leaving a portion of its top surface uncovered.

Performing step c can be done by any numerical simulation method known from the person skilled in the art. In embodiments, it is also possible to perform step c by an analytical method known from the person skilled in the art.

Preferably, step c is performed by a finite element method simulation. Examples of suitable software are Comsol, Agrosdd, and CST.

The predefined threshold depends on the materials used for the electrode and on the use of the electrode. In embodiments, the predefined threshold may be 0.7 A.cm⁻², preferably 0.6 A.cm⁻². This is advantageous as it avoids tissue damage when the electrode is used for in vitro cell interfacing or for brain stimulation.

There are two main embodiments to perform step f. In the first main embodiment, step f is performed by surrounding the electrically conductive element (1) provided in step a with a dielectric wall (2), by reducing the height of an existing wall (2) already surrounding the electrically conductive element (1) provided in step a, or by increasing the height of an existing wall (2) already surrounding the electrically conductive element (1) provided in step a.

Providing an electrically conductive element (1), identical to the electrically conductive element (1) provided in step a can, therefore, mean either actually reusing the electrically conductive element (1) or providing a new electrically conductive element (1) which is identical to the electrically conductive element (1) provided in step a. In the second main embodiment, step f is performed by repeating step a anew (in the same conditions), and surrounding the newly formed electrically conductive element (1) with a dielectric wall (2). The first main embodiment has the advantage that a wall (2) is built that is tailor-made for the actual electrically conductive element (1) that will form part of the electrode (3). The second main embodiment has the advantage that steps b to e of the method do not have to be repeated for each produced electrode (3) since step f can be repeated an indefinite number of times, each time with the same wall height (h) as determined, once and for all, in step e. This favours high throughput. Of course, in this last method, the wall height (h) is not tailor-made for the actual electrically conductive element (1) that will form part of the electrode (3). However, electrically conductive elements (1) produced in the same conditions are expected to be typically sufficiently similar for a same wall height (h), as determined in steps b to e, to be adequate and close to optimal for each electrically conductive element (1).

Typically, the wall height (h) of the dielectric wall (2) surrounding the electrically conductive element (1) provided in step f is measured from the bottom surface of the electrically conductive element (1) to a top surface of the wall (2) and is smaller than 0.1038 times, preferably smaller than 0.1000 times the length (or diameter) of that electrically conductive element (1). The length (or diameter) is measured parallel to the top surface of the substrate (5).

Typically, the dielectric wall (2) provided in step f is from 0.1 to 3 µm high.

Although the height of the electrode is relevant, the width of the wall, measured parallel to the top surface of the wall, is not relevant as it does not impact the current density profile of the electrode (3).

In embodiments, fabricating the dielectric wall (2) can be performed as follow (see Fig. 1 steps v and vi): First, the electrically conductive element (1) can be entirely embedded in a planar dielectric layer (see Fig. 1 v). Then, the planar dielectric layer can be patterned to expose the top surface of the electrically conductive element (1), while leaving in place a part of the dielectric layer in physical contact with the edges of the electrically conductive element (1), thereby forming the dielectric wall (2).

Entirely embedding the electrically conductive element (1) in a planar dielectric layer can be performed, for instance, by entirely covering the electrically conductive layer (12) with a dielectric layer (e.g. SiO₂), then planarizing the dielectric layer by chemical mechanical polishing.

The present disclosure also relates to an electrode (3) obtainable by a method according to any embodiment of the present invention, the electrode comprising an electrically conductive element (1) surrounded by a wall (2) which height is smaller than 0.1038 times, preferably smaller than 0.1000 times the length (or diameter) of the electrically conductive element (1), wherein said wall height (h) is measured from a bottom surface of the electrically conductive element (1) to a top surface of the wall (2). The length (or diameter) is measured parallel to the top surface of the substrate (5)

The present disclosure also relates to a brain stimulation system comprising such an electrode (3) and an electrical pulse generator electrically coupled to the electrode (3).

The present disclosure also relates to an array of said electrodes.

For example, the array of electrode (3) may be for in vitro cell interfacing.

The present disclosure also relates to a neural probe comprising said electrode (3) or the array of electrodes (3).

As used herein and unless provided otherwise, a neural probe is a device for implanting into the brain or other nervous tissues.

It is to be understood that although preferred embodiments, specific constructions and configurations, as well as materials, have been discussed herein, various changes or modifications in form and detail may be made without departing from the scope of the present invention which is defined by the appended claims.

## Claims

1. A method for manufacturing an electrode (3), comprising the steps of:
a. providing an electrically conductive element (1) over a top surface of a substrate (5),
b. measuring at least one parameter indicative of the shape or of the dimensions of the electrically conductive element (1),
c. simulating the electrically conductive element (1) and a dielectric wall (2) surrounding the same for a plurality of wall heights by using the at least one parameter as an input,
d. for each wall height, compute the maximum current density present at a surface of the electrically conductive element (1),
e. determine, from the maximum current densities computed in step d, one or more wall heights (h) for which the maximum current density is below a predefined threshold,
f. providing an electrically conductive element (1), identical to the electrically conductive element (1) provided in step a, surrounded by a wall (2) having a wall height (h) as determined in step e.

2. The method according to claim 1, wherein step e consists in identifying amongst the one or more wall heights (h) for which the maximum current density is below a predefined threshold, the wall height (h) giving rise to the lowest maximum current density, and wherein step f consists of providing an electrically conductive element (1), identical to the electrically conductive element (1) provided in step a, surrounded by a wall (2) having a wall height (h) closer to the wall height (h) identified in step e than to any other wall heights (h) amongst said one or more wall heights (h).

3. The method according to claim 2, wherein the brain stimulation is a deep brain stimulation.

4. The method according to any one of the preceding claims, wherein the electrically conductive element (1) provided in step a has a length measured parallel to the top surface of the substrate (5) which is larger than a height measured perpendicular to the top surface of the substrate (5).

5. The method according to any one of the preceding claims, wherein the electrically conductive element (1) provided in step a has a length measured parallel to the top surface of the substrate (5) which measures from 5 to 200 µm, preferably from 10 to 50 µm.

6. The method according to any one of the preceding claims, wherein the substrate (5) is a shank at least three times as heigh as it is wide.

7. The method according to any one of the preceding claims, wherein a top surface of the electrically conductive element (1) provided in step a has a concave shape with a concavity facing the substrate (5).

8. The method according to any one of the preceding claims, wherein the wall height of the wall (2) surrounding the electrically conductive element (1) provided in step f is measured from a bottom surface of the electrically conductive element (1) to a top surface of the wall (2) and is smaller than 0.1038 times, preferably smaller than 0.1000 times the length of that conductive element measured parallel to the top surface of the substrate (5).

9. The method according to any one of the preceding claims wherein the wall (2) surrounding the electrically conductive element (1) provided in step f has a wall height within 10% of a wall height minimizing maximum current density.

10. The method according to any one of the preceding claims, wherein the wall (2) provided in step f is from 0.11 to 3 µm high.

11. The method according to any one of the preceding claims, wherein a geometrical center of the top surface of the electrically conductive element (1) is higher than any edge of the top surface of that conductive element.

## Patentansprüche

1. Ein Verfahren zur Herstellung einer Elektrode (3), das die Schritte umfasst zum:
a.- Bereitstellen eines elektrisch leitfähigen Elements (1) über einer oberen Oberfläche eines Substrats (5),
b.- Messen mindestens eines Parameters, der die Form oder die Abmessungen des elektrisch leitfähigen Elements (1) angibt,
c.- Simulieren des elektrisch leitfähigen Elements (1) und einer dieses umgebenden dielektrischen Wand (2) für eine Vielzahl von Wandhöhen unter Verwendung des mindestens einen Parameters als Eingabe,
d.- Berechnen für jede Wandhöhe die maximale Stromdichte, die an einer Oberfläche des elektrisch leitfähigen Elements (1) vorhanden ist,
e.- Bestimmen aus den in Schritt d berechneten maximalen Stromdichten einer oder mehrerer Wandhöhen (h), für welche die maximale Stromdichte unter einem vordefinierten Schwellenwert liegt,
f.- Bereitstellen eines elektrisch leitfähigen Elements (1), das mit dem in Schritt a bereitgestellten elektrisch leitfähigen Element (1) identisch ist und von einer Wand (2) umgeben ist, die eine Wandhöhe (h) aufweist, wie sie in Schritt e bestimmt wurde.

2. Das Verfahren nach Anspruch 1, wobei Schritt e aus Identifizieren unter der einen oder mehreren Wandhöhen (h), bei denen die maximale Stromdichte unter einem vordefinierten Schwellenwert liegt, der Wandhöhe (h) besteht, welche die niedrigste maximale Stromdichte ergibt, und wobei Schritt f aus Bereitstellen eines elektrisch leitfähigen Elements (1) besteht, das mit dem in Schritt a bereitgestellten elektrisch leitfähigen Element (1) identisch ist, das von einer Wand (2) umgeben ist, die eine Wandhöhe (h) näher an der in Schritt e identifizierten Wandhöhe (h), als jede andere Wandhöhe (h) aus der einen oder mehreren Wandhöhen (h) aufweist.

3. Das Verfahren nach Anspruch 2, wobei die Hirnstimulation eine tiefe Hirnstimulation ist.

4. Das Verfahren nach einem der vorstehenden Ansprüche, wobei das in Schritt a bereitgestellte elektrisch leitfähige Element (1) eine parallel zur oberen Oberfläche des Substrats (5) gemessene Länge aufweist, die größer ist als eine senkrecht zur oberen Oberfläche des Substrats (5) gemessene Höhe.

5. Das Verfahren nach einem der vorstehenden Ansprüche, wobei das in Schritt a bereitgestellte elektrisch leitfähige Element (1) eine parallel zur oberen Oberfläche des Substrats (5) gemessene Länge von 5 bis 200 µm, vorzugsweise von 10 bis 50 µm aufweist.

6. Das Verfahren nach einem der vorstehenden Ansprüche, wobei das Substrat (5) ein Schaft ist, der mindestens dreimal so hoch wie breit ist.

7. Das Verfahren nach einem der vorstehenden Ansprüche, wobei eine obere Oberfläche des in Schritt a bereitgestellten elektrisch leitfähigen Elements (1) eine konkave Form mit einer zum Substrat (5) zeigenden Konkavität aufweist.

8. Das Verfahren nach einem der vorstehenden Ansprüche, wobei die Wandhöhe der Wand (2), die das in Schritt f bereitgestellte elektrisch leitfähige Element (1) umgibt, von einer unteren Oberfläche des elektrisch leitfähigen Elements (1) bis zu einer oberen Oberfläche der Wand (2) gemessen wird und kleiner ist als das 0,1038-fache, vorzugsweise kleiner als das 0,1000-fache der Länge dieses leitfähigen Elements, parallel zur oberen Oberfläche des Substrats (5) gemessen.

9. Das Verfahren nach einem der vorstehenden Ansprüche, wobei die Wand (2), die das in Schritt f bereitgestellte elektrisch leitfähige Element (1) umgibt, eine Wandhöhe innerhalb von 10 % einer Wandhöhe aufweist, welche die maximale Stromdichte minimiert.

10. Das Verfahren nach einem der vorstehenden Ansprüche, wobei die in Schritt f bereitgestellte Wand (2) 0,11 bis 3 µm hoch ist.

11. Das Verfahren nach einem der vorstehenden Ansprüche, wobei ein geometrischer Mittelpunkt der oberen Oberfläche des elektrisch leitfähigen Elements (1) höher liegt als jede Kante der oberen Oberfläche dieses leitfähigen Elements.

## Revendications

1. Un procédé de fabrication d'une électrode (3), comprenant les étapes de :
a. fournir un élément électriquement conducteur (1) sur une surface supérieure d'un substrat (5),
b. mesurer au moins un paramètre indicatif de la forme ou des dimensions de l'élément électriquement conducteur (1),
c. simuler ledit élément électriquement conducteur (1) et une paroi diélectrique (2) entourant ledit élément pour une pluralité de hauteurs de paroi en utilisant ledit au moins un paramètre comme entrée,
d. pour chaque hauteur de paroi, calculer la densité de courant maximale présente à une surface de l'élément électriquement conducteur (1),
e. déterminer, à partir des densités de courant maximales calculées à l'étape d, une ou plusieurs hauteurs de paroi (h) pour lesquelles la densité de courant maximale est inférieure à un seuil prédéfini,
f. fournir un élément électriquement conducteur (1), identique à l'élément électriquement conducteur (1) fourni à l'étape a, entouré d'une paroi (2) ayant une hauteur de paroi (h) telle que déterminée à l'étape e.

2. Le procédé selon la revendication 1, dans lequel l'étape e consiste à identifier parmi lesdites une ou plusieurs hauteurs de paroi (h) pour lesquelles la densité de courant maximale est inférieure à un seuil prédéfini, la hauteur de paroi (h) donnant lieu à la densité de courant maximale la plus basse, et dans lequel l'étape f consiste à fournir un élément électriquement conducteur (1), identique à l'élément électriquement conducteur (1) fourni à l'étape a, entouré d'une paroi (2) ayant une hauteur de paroi (h) plus proche de la hauteur de paroi (h) identifiée à l'étape e que de toute autre hauteur de paroi (h) parmi lesdites une ou plusieurs hauteurs de paroi (h).

3. Le procédé selon la revendication 2, dans lequel la stimulation cérébrale est une stimulation cérébrale profonde.

4. Le procédé selon l'une quelconque des revendications précédentes, dans lequel l'élément électriquement conducteur (1) fourni à l'étape a a une longueur mesurée parallèlement à la surface supérieure du substrat (5) qui est plus grande qu'une hauteur mesurée perpendiculairement à la surface supérieure du substrat (5).

5. Le procédé selon l'une quelconque des revendications précédentes, dans lequel l'élément électriquement conducteur (1) fourni à l'étape a a une longueur mesurée parallèlement à la surface supérieure du substrat (5) qui mesure de 5 à 200 µm, de préférence de 10 à 50 µm.

6. Le procédé selon l'une quelconque des revendications précédentes, dans lequel le substrat (5) est une tige au moins trois fois plus haute que large.

7. Le procédé selon l'une quelconque des revendications précédentes, dans lequel une surface supérieure de l'élément électriquement conducteur (1) fourni à l'étape a a une forme concave avec une concavité faisant face au substrat (5).

8. Le procédé selon l'une quelconque des revendications précédentes, dans lequel la hauteur de paroi de la paroi (2) entourant l'élément électriquement conducteur (1) fourni à l'étape f est mesurée depuis une surface inférieure de l'élément électriquement conducteur (1) jusqu'à une surface supérieure de la paroi (2) et est inférieure à 0,1038 fois, de préférence inférieure à 0,1000 fois la longueur de cet élément conducteur mesurée parallèlement à la surface supérieure du substrat (5).

9. Le procédé selon l'une quelconque des revendications précédentes, dans lequel la paroi (2) entourant l'élément électriquement conducteur (1) fourni à l'étape f a une hauteur de paroi dans les 10% d'une hauteur de paroi minimisant la densité de courant maximale.

10. Le procédé selon l'une quelconque des revendications précédentes, dans lequel la paroi (2) fournie à l'étape f a une hauteur de 0,11 à 3 µm.

11. Le procédé selon l'une quelconque des revendications précédentes, dans lequel un centre géométrique de la surface supérieure de l'élément électriquement conducteur (1) est plus haut que n'importe quel bord de la surface supérieure de cet élément conducteur.
